(19) Europäisches Patentamt
European Patent Office
Office européen des brevets

(11) Veröffentlichungsnummer: **0 000 015**
**B1**

(12) **EUROPÄISCHE PATENTSCHRIFT**

(21) Anmeldenummer: **78100027.8**

(22) Anmeldetag: **01.06.78**

(51) Int. Cl.³: **A 61 K 31/55**
A 61 K 31/55, 31/47

(54) **Arzneimittel zur Behandlung von Depressionen**

(30) Priorität: **01.06.77 DE 2724683**

(43) Veröffentlichungstag der Anmeldung:
**20.12.78 Patentblatt 78/01**

(45) Bekanntmachung des Hinweises auf die Patenterteilung:
**23.07.80 Patentblatt 80/15**

(84) Benannte Vertragsstaaten:
**BE CH DE FR GB NL SE**

(56) Entgegenhaltungen:
**ARZNEIM, FORSCH, *21,* Nr. 7, Seite 1045 (1971)
"8-Amino-Phenyl-1,2,3,4-tetrehydroiso-
chinoline, eine neue Gruppe antidepressiver
Psychopharmaka"**

(73) Patentinhaber: **Hoechst Aktiengesellschaft
Postfach 80 03 20
D - 6230 Frankfurt
Main 80 (DE)**

(72) Erfinder: **Schmitt, Karl, Dr.
Hasenpfad 3
D - 6232 Bad Soden am Taunus (DE)
Hoffmann, Irmgard, Dr.
Ovanienstrasse 1
D - 6232 Bad Soden am Taunus (DE)
Fülberth, Werner, Dr.
Theodor-Storm-Strasse 11
D - 6233 Kelkheim (Taunus) (DE)
Stammberger, Willi, Dr.
Sachsenring 15
D - 6238 Hofheim am Taunus (DE)**

**EP 0 000 015 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Arzneimittel zur Behandlung von Depressionen

Die Erfindung betrifft ein Arzneimittel zur Behandlung von Depressionen, das durch einen Gehalt an einer Verbindung der Formel I

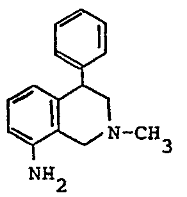

I

oder deren Säuren (Wirkstoff A) und 7-Chlor-1-methyl - 5 - phenyl - 1H - 1,5 - benzodiazepin-2,4-(3H,5H)-dion (Wirkstoff B) gekennzeichnet ist.

Die als Wirkstoff A verwendete Verbindung ist aus DBP 1 670 694 bekannt und trägt den Namen "Nomifensin" (INN).

Nomifensin wird in Form des Hydrogenmaleinates als Arzneimittel für die Therapie depressiver Zustände eingesetzt. Es ist das erste stark wirksame Antidepressivum, das sich nicht von einem tricyclischen Ringsystem ableitet. Im Gegensatz zu den bekannten tricyclischen Antidepressiva ist Nomifensin frei von ernsthaften Nebenwirkungen, vergl. z.B. "Arzneimittelforschung", *23,* (1973), 45—50.

Bei Depressionen, die mit ausgeprägten Angstzuständen einhergehen, hat es sich als günstig erwiesen, neben der Ba sismedikation mit Nomifensin eine Begleittherapie mit einem ausgesprochen anxiolytisch wirkenden Präparat durchzuführen.

Für die anxiolytische Begleittherapie hat sich das 7 - Chlor - 1 - methyl - 5 - phenyl - 1H - 1,5-benzodiazepin - 2,4 - (3H, 5H) - dion bewährt, das unter dem Namen Clobazam (INN) bekannt geworden ist (vergl. z.B. La Chimica e l'Industria *51,* (1969), 479—483).

Es hat sich nun als vorteilhaft herausgestellt, die beiden Wirkstoffe A und B in einem pharmazeutischen Präparat zu kombinieren. Die Verwendung dieses Kombinationspräparates bedingt nicht nur eine Vereinfachung der Medikation, sondern es hat sich darüber hinaus gezeigt, daß überraschenderweise von den kombinierten Wirkstoffen für den gleichen Therapieerfolg geringere Mengen erforderlich sind als bei separater Medikation.

Der Wirkstoff A ist eine basische Verbindung, die in den meisten Fällen vorteilhaft in Form eines physiologisch verträglichen Salzes verwendet wird. Für Salze in Betracht kommende Säuren sind z.B. Chlor-, Brom- und Jodwasserstoffsäure, Phosphorsäure, Schwefelsäure, Amidosulfonsäure, Methylschwefelsäure, Salpetersäure, Ameisensäure, Essigsäure, Propionsäure, Bernsteinsäure, Weinsäure, Milchsäure, Malonsäure, Fumarsäure, Zitronensäure, Äpfelsäure, Schleimsäure, Benzoesäure, Salicylsäure, Acetursäure, Embonsäure, Naphthalin-1,5 - disulfonsäure, Ascorbinsäure, Hydroxyäthansulfonsäure, Benzolsulfonsäure, oder auch synthetische Harze, die saure Gruppen enthalten.

Der Wirkstoff B ist eine neutrale Verbindung, die in Wasser schwer löslich ist. Clobazam wird daher vorteilhaft in mikronisierter Form verwendet.

Die erfindungsgemäße Wirkstoffkombination kann in den üblichen Anwendungsformen wie Tabletten, Pulver, Kapseln, Suppositorien oder Dragees zur Anwendung kommen. Vorzugsweise verwendet man Kapseln, Tabletten und Dragees. Es werden die üblichen pharmazeutischen Hilfsstoffe, beispielsweise inerte Verdünnungsmittel und Bindemittel wie Milchzucker, mikrokristalline Zellulose, Calciumcarbonat, Di- und Tricalciumphosphat, Polyäthylenglykol 4 000 — 6 000, Gelatine, Stärkeschleim, Sprengmittel wie Stärken, Ultraamylopektin, Zellulose und Zellulosederivate, Aerosil, Schmiermittel wie Talkum, Magnesiumstearat, Calciumstearat, Stearinsäure hinzugefügt.

Als Tabletten können auch solche eingesetzt werden, die aus mehreren Schichten bestehen. Ihre Herstellung erfolgt beispielsweise durch Mischen der Wirkstoffe mit den pharmazeutischen Hilfsstoffen nach üblichen galenischen Fertigungsmethoden.

Dragees können beispielsweise durch Überziehen von Kernen mit üblicherweise in Drageeüberzügen verwendeten Mitteln, z.B. Rohrzucker, Gelatine, Gummi arabicum, Talkum, Calciumkarbonat, Cera alba, Cera Carnauba, Aerosil erhalten werden. Die Herstellung von Kapseln erfolgt ebenfalls in an sich bekannter Weise, wobei inerte Träger wie Maisstärke, Talkum, Aerosil gemischt werden und vorzugsweise Gelatinekapseln Verwendung finden. Auch in Form von Zäpfchen kann die erfindungsgemäße Kombination Einsatz finden, wobei die üblichen Trägermittel wie Triglyceride gesättigter Fettsäuren, hydrierte pflanzliche Fette zuzugeben sind.

Die bevorzugte Applikationsform für das erfindungsgemäße antidepressiv wirksame pharmazeutische Präparat ist die orale Gabe. Hierzu kommt der Wirkstoff A in einer Dosis von 5 bis 50 mg pro Verabreichungseinheit in Betracht. Von Clobazam, dem Wirkstoff B, werden 2 bis 25 mg verwendet. In Fällen, die eine höhere Dosierung erfordern, kann ein entsprechendes Mehrfaches der vorstehend genannten Dosierungen verabfolgt werden.

Ausführungsbeispiele:

Kapseln

| | | |
|---|---|---|
| 1. Nomifensin-Hydrogen-maleinat | 50 mg | 25 mg |
| 2. Clobazam | 15 mg | 7.5 mg |
| 3. Maisstärke | 35 mg | 17.5 mg |
| 4. Mikrokristalline Cellulose | 80 mg | 40 mg |
| 5. Talkum | 10 mg | 5 mg |
| 6. Hochdisperses Siliciumdioxid | 6 mg | 3 mg |
| 7. Magnesiumstearat | 4 mg | 2 mg |
| | 200 mg | 100 mg |

Die mikronisierten Wirkstoffe 1. und 2. werden mit den Zuschlägen 3.—7. homogen gemischt. Die Mischung wird in bekannter Weise in Hartgelatine-Steckkapseln abgefüllt.

Tabletten

| | | |
|---|---|---|
| 1. Nomifensin | 25 mg | 50 mg |
| 2. Clobazam | 7,5 mg | 15 mg |
| 3. Lactose | 40 mg | 80 mg |
| 4. Maisstärke | 24 mg | 48 mg |
| 5. Mikrokristalline Cellulose | 20 mg | 40 mg |
| 6. Hochdisperses Siliciumoxid | 3 mg | 6 mg |
| 7. Polyvinylpyrrolidon K 25 | 6,5 mg | 13 mg |
| 8. Talkum | 3,5 mg | 7 mg |
| 9. Magnesiumstearat | 0,5 mg | 1 mg |
| | 130 mg | 260 mg |

Die Tabletten-Mischung aus den Substanzen 1.—9. wird granuliert und zu bikonvexen Tabletten verpresst.

*Filmlacktabletten*
Die Tabletten werden mit einem der üblichen Filmlacke überzogen.

*Dragees*
Das zur Herstellung von Tabletten angefertigte Granulat wird zu Drageekernen verpreßt. Die Drageekerne werden in bekannter Weise dragiert.

**Patentanspruch**

1. Arzneimittel zur Behandlung von Depressionen, gekennzeichnet durch einen Gehalt an einer Verbindung der Formel I

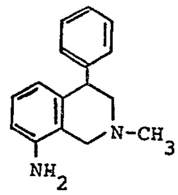

I

oder deren Salze (Wirkstoff A) und einem Gehalt an 7-Chlor-1-methyl-5-phenyl-1H-1,5-benzodiazepin-2,4-(3H, 5H)-dion (Wirkstoff B).

**Revendication**

1. Médicament pour le traitement des dépressions, caractérisé en ce qu'il contient un composé de formule I

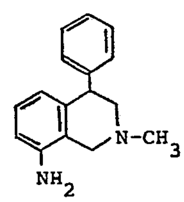

I

ou ses sels avec des acides (substance active A) et la 7-chloro-1-méthyl-5-phényl-1H-1,5-benzodiazépine-2,4-(3H, 5H)-dione (substance active B).

**Claim**

1. Medicament for the treatment of depressions characterized by a content of a compound of formula I

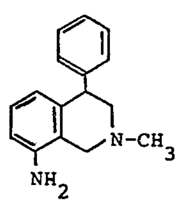

I

or of its salts with acids (active substance A) and of 7-chloro-1-methyl-5-phenyl-1H-1,5-benzodiazepine-2,4-(3H, 5H)-dione (active substance B).